# EUROPEAN PATENT APPLICATION

(11) **EP 1 030 245 A2**
(43) Date of publication of application: **23.08.2000**
(21) Application number: 99125640.5
(22) Date of filing: 22.12.1999
(51) Int. Cl.: G06F 15/02

(54) **A device for determining nutritional data of food eaten by a person**

(30) Priority: 29.12.1998 IT TO981091
(71) Applicant: Nevenka, Gregoric, 10098 Rivoli (Torino) (IT)
(72) Inventor: Nevenka, Gregoric, 10098 Rivoli (Torino) (IT)
(74) Representative: Marchitelli, Mauro

(57) **Abstract**

A device for determing nutritional data of food eaten by a person, comprising:
- a keyboard (10) for manually controlling the operation of the device,
- a base memory (24) in which there are stored nutritional data of a series of food elements,
- an alphanumeric display (12) for displaying information relating to the type of element and to its nutritional features, and
- a microprocessor control unit (26) programmed for enabling the selection by means of the keyboard (10) of individual elements among the ones stored in the base memory and for displaying on said display (12) the nutritional data of the selected element.

After the selection of a food element among the ones stored in the base memory (24), the control unit (26) is prepared for receiving information relating to the quantity of such element which has been eaten or which is intended to be eaten and for calculating the total nutritional data due to said quantity of the selected element. The control unit (26) is also programmed for adding said total nutritional data to a resettable progressive memory (30) containing the sum of total nutritional data of food elements eaten in a period of time subsequent to the last reset.

## Description

The present invention relates to a device for determining nutritional data of food eaten by a person.

The object of the present invention is to provide a pocket device which is simple to use and permits to control nutritional data of food eaten by a person during a period of time, for instance during one day.

According to the present invention, this object is achieved by a device having the features forming the subject of the claims.

A preferred embodiment of the invention will now be disclosed in detail with reference to the annexed figure, given purely by way of non-limiting example, which shows schematically the operation of a device according to the invention.

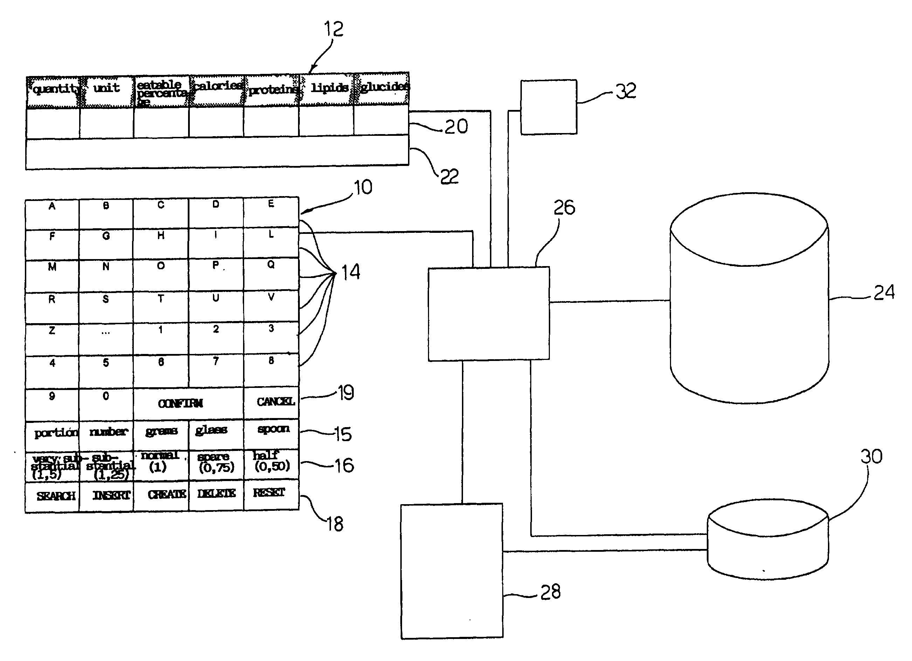

The device according to the invention can be produced with the general configuration of a battery-powered pocket calculator, with dimensions and weigth such that it can be contained in the pocket of a shirt. With reference to the drawing, the device comprises a keyboard 10 and a display 12. On the keyboard there are provided a series of alphanumeric keys indicated alltogether at 14, a series of keys 15 for defining the unit of food, a series of keys 16 for defining the quantity of food, a series of function keys 18 and a pair of keys 19 for confirming or cancelling the input data.

The display 12 has an alphanumeric control field and seven alphanumeric fields 22 intended to display, respectively, information relating to: reference quantity of food (quantity), unit, eatable percentage, and content of calories, proteins, lipids and glucides.

A base memory 24 stores nutritional data of the most common food. The memory 24 is organised as a table in which, for each type of food, there is indicated: reference quantity, unit, eatable percentage, and content of calories, proteins, lipids and glucides relating to the reference quantity of the food. The table is ordered on the basis of the description of the food. Preferably, data of the most common food-staff are already stored in the memory 24 and the user can store therein personalised food. The memory 24 is preferably of a permanent type, so that it can retain data also without power.

The device comprises a microprocessor control unit 26 programmed for carring out the functions which will be disclosed in the following. The device comprises also a temporary memory 28 and a progressive resettable memory 30, as well as an on-off switch 32.

The operation of the device according to the present invention is the following.

When the device is turned on, it displays the daily progressive amounts stored in the progressive memory 30. The display 12 shows separately the data contained in the progressive memory 30 relating to the total amount of calories and calories due to proteins, lipids and glycyls. Preferably, in addition to the amount of calories due to proteins, lipids and glucides, it is also displayed the percentage value of the respective data calculated on the total amount of calories.

By depressing the search key, the control unit 26 activates a function for searching in the base memory 24 the food corresponding to the definition input by the user. On the control line 20 of the display 12 there appear the letters which the user selects by means of the keys 14 and which define the food element to be searched. The search function is activated by pressing the key CONFIRM key. The control unit 26 searches in the base memory 24 with a conventional algoritm and displays on the display 22 the found definition which is closer to the definition digited by the user. If the definition which appears on the control line 20 corresponds to the searched food, the usor presses the key CONFIRM which displays the complete data of the selected food, comprising the reference quantity, unit, the eatable percentage and the content of calories, proteins, lipids and glucides corresponding to the reference quantity of the food. If the definition of the food appearing on the control line after the search does not correspond to the searched food, the user has either the possibility of carring out a new search or to scroll the definitions of food contained in the base memory in alphabetical order by using to scroll keys (UP and DOWN).

After having selected the desired food by pressing the key CONFIRM, the control unit 26 prepares itself for receiving from the keyboard 10 information relating to the quantity of the selected food. The user must insert the quantity of food which has eaten or which intends to eat, having regard to the food unit. Depending on the type of food, the unit can be "portion", "number", "grams", "glass", or "spoon". The indication of the quantity of eaten food can be carried out by digiting the number of food unit and then by pressing the key CONFIRM. For instance, if the food unit is "grams" the usor must digit the number corresponding to the quantity of eaten grams and then confirm the input by the key CONFIRM. As an alternative, the user can select the quantity by means of the quantity keys 16 which define the food unit as: very substantial, substantial, normal, spare, or half, which multiply the reference quantity by coefficients respectively equal to 1.5, 1.25, 1, 0.75 and 0.5. Therefore, if for instance the user selects a food whose unit is "portion", the quantity can be defined by pressing one of the keys 16 which qualify the quantity with reference to a normal avarage value. The keys 16 can also be used for indicating the quantity of food whose unit is "number", "glass", "spoon" or "grams"

By pressing the key CONFIRM after having defined the food quantity, the control unit 26 calculates the total nutritional data corresponding to the indicated quantity, taking into acount also the eatable percentage of the food. The display shows the quantity of food and the total contant of calories, proteins, lipids and glucides of the food corresponding to the selected quantity and to the respective eatable percentage. The displayed data are stored in the temporary memory 28 and the user has the possibility of either confirming or cancelling these data. If the data are confirmed, the content of the temporary memory 28 is added to the progressive memory 30 and there are displayed the progressive amounts of the nutritional data of the eaten food. On the contrary, a pressure on the key CANCEL removes the nutritional data from the temporary memory, without modifing the content of the progressive memory 30. The progressive memory 30 can be reset at the end of a reference period (for instance at the end of each day).

During the previously disclosed procedure, the control line 20 of the display 12 shows in each turn instructions requesting, far instance, the definition of the food to be searched, the quantity of the selected food and to confirm the addition of the total nutritional data of a certain food to the progressive memory.

The data contained in the base memory 24, can be personalised by the functions INSERT or CREATE. The function INSERT permits to store in the base memory 24 a new food for which it would be necessary to input: description, reference quantity, unit, eatable percentage and content of calories, proteins, lipids and glucides for the reference unit. Also in this case, explanations or instruction which facilitates the input procedure appear on the control line 20.

The function CREATE differs from the function INSERT in that it permits to define nutritional data of a new food starting by elements already stored in the base memory 24. The function CREATE activates a plurality of search functions through which the base elements of a new recipe to be created are selected and the respective quantity are indicated. When the composition of the recipe is completed, the nutritional data of the new food with the respective definition are stored in the base memory 24.

The user has also the possibility of eliminating food elements stored in the base memory 24 in order to either free memory space or to simplify the search by means of the scroll keys.

## Claims

1. A device for determining nutritional data of food eaten by a person, characterised in that it comprises:
- a keyboard (10) for manually controlling the operation of the device,
- a base memory (24) in which there are stored nutritional data of a series of food elements,
- an alphanumeric display (12) for displaying information relating to the type of food and its nutritional features, and
- a microprocessor control unit (26) programmed for enabling the selection by means of the keyboard (10) of individual elements among the ones stored in the base memory and for displaying on said display (12) the nutritional data of the selected element,
wherein, after the selection of an element among the ones stored in said base memory (24), said control unit (26) is prepared for receiving information relating to the quantity of such food which has been eaten or which is intended to be eaten and for calculating the total nutritional data due to said quantity of the selected food, the control unit (26) being also programmed for adding said total nutritional data to a resettable progressive memory (30) containing the sum of total nutritional data of food eaten in the period of time subsequent to the last reset.

2. A device according to claim 1, characterised in that it further comprises a temporary memory (28) in which there are temporarily stored said total nutritional data of the quantity of the selected food which has been eaten or which is intended to be eaten, before said total nutritional data are added to the content of the progressive memory (30).

3. A device according to claim 1, characterised in that said base memory (24) contains, for each element, information relating to a reference quantity of such element and to the nutritional content of the element relating to such reference quantity.

4. A device according to claim 3, characterised in that the nutritional content is displayed as contant of total calories and calories due to proteins, lipids and glucides of said reference quantity.

5. A device according to claim 4, characterised in that the content of calories due to proteins, lipids and glucides is displayed as absolute value and/or as percentage value calculated on the total value of calories.

6. A device according to claim 1, characterised in that said microprocessor control unit (26) is programmed for storing in the base memory (24) nutritional data relating to new food elements.

7. A device according to claim 1, characterised in that said microprocessor control unit (26) is programmed for storing in the base memory (24) new food elements whose nutritional data are obtained by summing with each other nutritional data of a plurality of elements whose nutritional data where previously contained in the base memory (24).

8. A device according to claim 1, characterised in that the keyboard (10) is provided with a series of keys (15) for defining the unit of food elements.

9. A device according to claim 1, characterised in that the keyboard (10) is provided with a series of keys (16) for defining predetermined quantities of food with reference to a predetermined unit.
